# EUROPEAN PATENT APPLICATION

(11) **EP 4 019 003 A1**
(43) Date of publication of application: **29.06.2022**
(21) Application number: 22151084.5
(22) Date of filing: 11.09.2014
(51) Int. Cl.: A61K 9/00, A61K 9/20, A61K 31/522, A61P 3/10

(54) **PHARMACEUTICAL FORMULATIONS OF LINAGLIPTIN**

(30) Priority: 12.09.2013 TR 201310724
(62) Divisional of application: 14184462.1
(71) Applicant: SANOVEL ILAC SANAYI VE TICARET A.S., Istanbul 34460 (TR)
(72) Inventor: TÜRKYILMAZ, Ali, Istanbul (TR); TURP, Ali Hasan, Istanbul (TR); SAYDAM, Mehtap, Istanbul (TR)
(74) Representative: Sevinç, Erkan

(57) **Abstract**

A pharmaceutical formulation for therapeutically effective amount of linagliptin or pharmaceutically acceptable salt thereof comprising croscarmellose sodium and at least one other pharmaceutically acceptable excipient.

## Description

### Field of Invention

A pharmaceutical formulation for therapeutically effective amount of linagliptin or pharmaceutically acceptable salt thereof comprising croscarmellose sodium and at least one other pharmaceutically acceptable excipient.

### Background of Invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

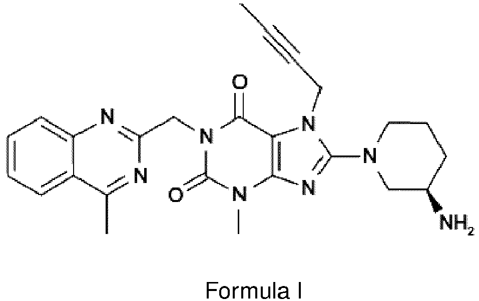

Its chemical name is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

There is Linagliptin coated tablet formulation license in the US and EU under the brand name TRADJENTA^{®} by Boehringer Ingelhem International. TRADJENTA^{®} includes mannitol, prejelatinized starch, maize starch, copovidone, magnesium stearate in the tablet core and hypromellose, titanium dioxide, talc, macrogol 6000 and red iron oxide in the film coating.

It is used once daily and can be used either alone or in combination with insulin or other glycemic agents. The currently commercially available dose of Linagliptin is 5 mg and due to the low active substance content in the final formulation, the active substance is milled in order to ensure an adequate content uniformity. Contrary to commercially available dose of Linagliptin, in this present invention, to obtain desired content uniformity, croscarmellose sodium was used as a disintegrant.

EP 2 023 902 B1 provides pharmaceutical formulations comprising DPP-4 inhibitors included Linagliptin. It is indicated that DPP-4 inhibitors with primary amine group shows incompatibilities, degradation problems or extraction problems with excipients such as microcrystalline cellulose, sodium starch glycolate, croscarmellose sodium, tartaric acid, citric acid, glucose, fructose, saccharose, lactose, maltodextrines. Linagliptin has also a primary amine group on its chemical structure. In solid dosage forms, it may react with many excipients or impurities of excipients, although linagliptin itself is very stable. Thus, aforementioned patent provides Linagliptin formulations which do not comprise above excipients included croscarmellose sodium.

In this invention, croscarmellose sodium has been used as a disintegrant to achieve required content uniformity in the solid dosage form of linagliptin. To overcome stability problem if may arise from croscarmellose, at least one other pharmaceutically acceptable excipient has been used.

### Description of the invention

A pharmaceutical formulation for therapeutically effective amount of linagliptin or pharmaceutically acceptable salt thereof comprising croscarmellose sodium and at least one other pharmaceutically acceptable excipient.

In one embodiment, linagliptin or a pharmaceutically acceptable salt thereof is present in an amount of 2 to 80 %, preferably 3.5 to 60 % and more preferably 10 to 40 % by weight of total formulation.

It is known that obtaining an adequate content uniformity is an important issue for pharmaceutical solid dosage forms during the process, because it is difficult to disintegrate active ingredients homogenously, especially if they have low doses of active agent. Moreover, inadequate content uniformity results undesired side effects and low bioavailability during the therapy. In this invention, to obtain desired content uniformity, croscarmellose sodium was used as a disintegrant. Without a milling process of linagliptin as in state of art, desired content uniformity has been achieved by using croscarmellose sodium.

In one embodiment, the amount of croscarmellose sodium is in the range of 3 to 50 %, preferably 6 to 25 %, more preferably it is 10 to 20 % by weight of total formulation.

According to this invention, the final dosage form of the pharmaceutical formulation of linagliptin has a content uniformity of less than 3.0 % RSD (Relative Standard Deviation), preferably it has a content uniformity of less than 2.0 % RSD. Herein, content uniformity test is determined by calculating RSD (Relative Standard Deviation) value of 10 tablets and RSD value is determined as described in pharmacopoeia USP31-NS26S2, chapter 905.

According to one embodiment, said at least one other pharmaceutically acceptable excipient is selected from the group comprising disintegrants, fillers, binders, lubricants and glidants.

In this embodiment, suitable disintegrants may include, but not limited to alginic acid and alginates, ion-exchange resins, magnesium aluminum silica, sodium dodecyl sulphate, sodium carboxy methyl cellulose, carboxy methyl cellulose calcium, polyvinylpyrrolidone, docusate sodium, guar gam, low-substitue HPC, HPMC, polyacrylin potasium, poloxomer, sodium alginate, sodium glysin carbonate, sodium lauryl sulphate.

In one embodiment, suitable fillers may include but not limited to dibasic calcium phosphate, tribasic calcium phosphate, sorbitol, sucrose, trehalose, isomalt, microcrystalline cellulose, mannitol, lactose, starch, sodium carbonate, sodium bicarbonate, dextrose, maltodextrine, izomalt, calcium carbonate, xylitol or mixtures thereof, preferably dibasic calcium phosphate.

In general, DPP-4 inhibitors are not very stable compounds. Especially, in solid dosage forms, amine group containing DPP-4 inhibitors like linagliptin may react with many excipients or impurities of excipients. In prior art, croscarmellose sodium has been indicated as a reactive substance with amine group containing DPP-4 inhibitors. In this invention, it has been surprisingly found that using dibasic calcium phosphate ensures the stability of linagliptin in a solid dosage formulation. Furthermore, dibasic calcium phosphate gives good flow and compaction properties to the formulation due to its bulk density.

The stability assay is performed by HPLC at a wavelength of 225nm at 45 ºC in the presence of phosphate buffer.

According to this embodiment, the amount of dibasic calcium phosphate is in the range of 2 to 90 % by weight of total formulation, preferably 10 to 75 % by weight of total formulation, more preferably 30 to 50 % by weight of total formulation.

In one embodiment, the ratio of dibasic calcium phosphate to croscarmellose sodium is in the range of 2 to 20 (w/w) and preferably 5 to 15 (w/w).

In one embodiment, suitable binders may include but not limited to sugars, glycose syrups, natural gums, guar gum, gelatins, pullulan, polymetacrylates, kollagen, agar, algynate, sodium alginate, hyaluronic acid, pectin, tragakanti gum, carboxymethyl cellulose, polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide and mixtures thereof.

According to one embodiment, suitable lubricants may include but not limited to sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin and mixtures thereof.

According to further embodiment, the present invention relates to a linagliptin solid dosage formulation does not comprise magnesium stearate in contrast to prior art. Magnesium stearate is a metal derivative lubricant. It has anti-adhesive and flow enhancement properties by ensuring uniformity of tablets. Besides this advantageous, its hydrophobic properties has disadvantages on the process such as prolonged mixing. Because of this increase in time, lubricant forms a hydrophobic film around the active agent and retards the dissolution by retarding wetting of active agent as well as decreases the compatibility of the powder mixture.

In this invention, sodium stearyl fumarate is selected as a suitable lubricant in tableting. Due to its hydrophilic properties, formulation does not have the disadvantages of magnesium stearate in respect of tablet compatibility, disintegration and dissolution.

According to this embodiment, the pharmaceutical formulation comprises one of said hydrophilic lubricants which are selected from sodium stearyl fumarate, polyethylene glycol, sodium benzoate or mixture thereof and said formulation does not comprise magnesium stearate.

The amount of sodium stearyl fumarate is present in the range of 0.01 to 10 %, preferably 0.1 to 5%, and more preferably 0.25 to 2.0 % by weight of total formulation.

The further advantage of the formulation with croscarmellose and dibasic calcium phosphate is to further improve disintegration and dissolution of linagliptin by using sodium stearyl fumarate without using magnesium stearate that cause undesirable dissolution profile.

According to these embodiments, the formulation wherein 75 to 90 % of the total amount of linagliptin by weight relative to the total weight of said formulation is dissolved in 45 minutes, preferably 30 minutes, more preferably in 15 minutes when measured in 900mL of 0.1N HCI at 50 rpm named as USP Apparatus I (Basket).

Suitable glidants may include but not limited to colloidal silicon dioxide, aluminium silicate and the like or mixtures thereof.

Coating may also preferably be used for moisture protection. It can be selected from the group comprising Polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA), and all kinds of OpadryTM, as well as pigments, dyes, titanium dioxide and iron oxide, talk and polymethylmetacrylate copolymers (Eudragit).

In this present invention, to achieve desired content uniformity and stability with an improved process, these formulations have been designed, comprising the following:
a. 2.0 - 80.0 % by weight of linagliptin
b. 3.0 - 50.0 % by weight of croscarmellose sodium
c. 2.0 - 90.0 % by weight of dibasic calcium phosphate
d. 0.01 -10.0 % by weight of sodium stearyl fumarate
e. 0.1 - 25.0 % by weight of pullulan
f. 0.1 - 3 % by weight of silicon dioxide
g. 0.2 - 10 % by weight of coating

a. 2.0 - 80.0 % by weight of linagliptin
b. 3.0 - 50.0 % by weight of croscarmellose sodium
c. 2.0 - 90.0 % by weight of dibasic calcium phosphate
d. 0.01 -10.0 % by weight of sodium stearyl fumarate
e. 0.1 - 3 % by weight of silicon dioxide
f. 0.2 - 10 % by weight of coating
and
a. 2.0 - 80.0 % by weight of linagliptin
b. 3.0 - 50.0 % by weight of croscarmellose sodium
c. 2.0 - 90.0 % by weight of dibasic calcium phosphate
d. 0.01 -10.0 % by weight of sodium stearyl fumarate
e. 0.1 - 25.0 % by weight of pullulan
f. 0.1 - 3 % by weight of silicon dioxide
g. 0.2 - 10 % by weight of coating

### Example 1: Wet Granulation

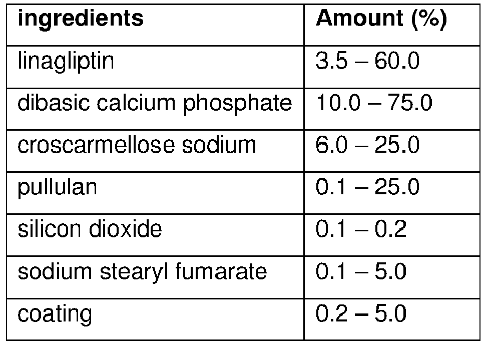

The production of the formulation is carried out as follows: Linagliptin, croscarmellose sodium and dibasic calcium phosphate are mixed and granulated with alcoholic/hydroalcoholic pullulan solution. Then, granules are dried and sieved. They are mixed with silicon dioxide and sodium stearyl fumarate respectively, and then pressed into tablets. Pressed tablets are preferably coated with PVA (polivinil alkol) based coatings such as Opadry AMB/ Kollicoat IR.

### Example 2: Pellet - Capsule

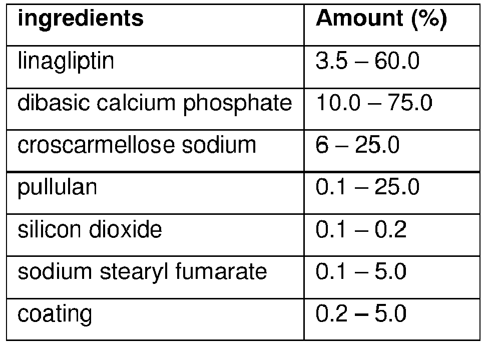

The production of the formulation is carried out as follows: Linagliptin, silicon dioxide, croscarmellose sodium and dibasic calcium phosphate are mixed and granulated with alcoholic/hydroalcoholic pullulan solution. Granules are passed through the extruder and pellets are obtained by spheronization. Pellets are mixed with sodium stearyl fumarate and filled into capsules or pressed into tablets. Pressed tablets are preferably coated with PVA (polivinil alkol) based coatings such as Opadry AMB/ Kollicoat IR.

### Example 3: Sugar Pellet - capsule

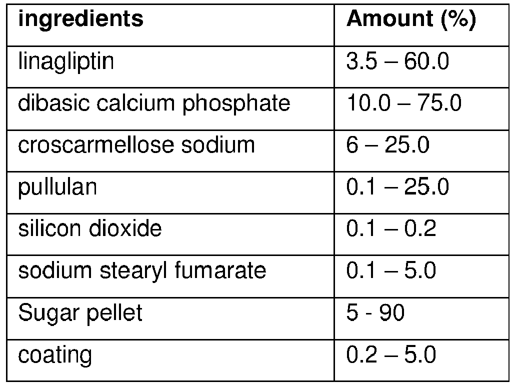

The production of the formulation is carried out as follows: The alcoholic/hydroalcoholic solution of Linagliptin with pullulan is prepared. Sugar pellets are coated with this solution. Pellets are mixed with croscarmellose sodium, dibasic calcium phosphate, silicon dioxide and sodium stearyl fumarate, respectively. Then, the mixture filled into capsules or pressed into tablets. Pressed tablets are preferably coated with PVA (polyvinyl alcohol) based coatings such as Opadry AMB/ Kollicoat IR.

With this invention, a pharmaceutical formulation comprising linagliptin or a pharmaceutically acceptable salt thereof is achieved which is eliminating disintegration, stability and process related problems and bringing additional advantages.

## Claims

1. A pharmaceutical formulation for linagliptin or a pharmaceutically acceptable salt thereof is present in an amount of 2 to 80 % and sodium stearyl fumarate as lubricant.

2. The pharmaceutical formulation according to claim 1, wherein linagliptin or a pharmaceutically acceptable salt thereof is present in an amount of 3.5 to 60 % and more preferably 10 to 40 % by weight of total formulation.

3. The pharmaceutical formulation according to claim 1, wherein the amount of sodium stearyl fumarate is in the range of 0.01 to 10 %, preferably 0.1 to 5%, and more preferably 0.25 to 2.0 % by weight of total formulation.

4. The pharmaceutical formulation according to claim 1, wherein further comprising at least one other pharmaceutically acceptable excipient which is selected from the group comprising disintegrants, fillers, binders and glidants.

5. The pharmaceutical formulation according to claim 4, wherein disintegrants may include, but not limited to alginic acid and alginates, ion-exchange resins, magnesium aluminum silica, sodium dodecyl sulphate, sodium carboxy methyl cellulose, carboxy methyl cellulose calcium, polyvinylpyrrolidone, docusate sodium, guar gam, low-substitue HPC, HPMC, polyacrylin potasium, poloxomer, sodium alginate, sodium glysin carbonate, sodium lauryl sulphate.

6. The pharmaceutical formulation according to claim 4, wherein the filler is selected from the group comprising dibasic calcium phosphate, tribasic calcium phosphate, trehalose, isomalt, microcrystalline cellulose, mannitol, lactose, starch, sodium carbonate, sodium bicarbonate, calcium carbonate or mixtures thereof.

7. The pharmaceutical formulation according to claim 4, wherein the binders may include but not limited to sugars, glycose syrups, natural gums, guar gum, gelatins, pullulan, polymetacrylates, collagen, agar, algynate, sodium alginate, hyaluronic acid, pectin, tragakanti gum, carboxymethyl cellulose, polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide and mixtures thereof.

8. The pharmaceutical formulation according to claim 4, wherein glidants may include but not limited to colloidal silicon dioxide, aluminium silicate and the like or mixtures thereof.
